# EUROPEAN PATENT APPLICATION

(11) **EP 2 465 439 A1**
(43) Date of publication of application: **20.06.2012**
(21) Application number: 12156892.7
(22) Date of filing: 14.08.2007
(51) Int. Cl.: A61B 8/12, A61B 8/08, A61B 18/14, A61B 19/00

(54) **Imaging device, imaging system, and methods of imaging**

(30) Priority: 14.08.2006 US 837320 P
(62) Divisional of application: 07811308.1
(71) Applicant: Volcano Corporation, San Diego, CA 92130 (US)
(72) Inventor: Magnin, Paul A., Andover, MA Massachusetts 01810 (US); Tse, Chen Fong, Calgary, Alberta T2T OT2 (CA); Bowden, Russell W., Tyngsboro, MA Massachusetts 01879 (US); Goodnow, John W., Arlington, MA Massachusetts 02474 (US); Miller, David G., Bradford, MA Massachusetts 01835 (US)
(74) Representative: Jansen, Cornelis Marinus

(57) **Abstract**

An imaging device, imaging system, and methods of imaging are provided. The imaging device may fit inside a diagnostic or therapeutic assembly, such as a biopsy, drainage, or other type therapy needle assembly. The imaging device may reside inside the diagnostic or therapy assembly as the combined device is advanced to a desired location. Two-dimensional or three-dimensional ultrasonic images may be produced that allow for the accurate placement of the diagnostic or therapy assembly.

## Description

### BACKGROUND

This application claims priority to U.S. Provisional Application No. 60/837,320 filed August 14, 2006, which is herby incorporated in reference in its entirety.

### 1. Field

An Imaging device, Imaging System, and Methods of Imaging are disclosed herein. 2. Background

Imaging devices, imaging systems, and methods of imaging are known. However, such known imaging assemblies, imaging systems, and methods of imaging suffer from various disadvantages.

### BRIEF DESCRIPTION OF THE DRAWINGS

Embodiments will be described in detail with reference to the following drawings in which like reference numerals refer to like elements, and wherein:

Figure 1 is a schematic block diagram of an imaging system according to an embodiment;

Figure 2A is a cross-sectional side view of an imaging device according to an embodiment;

Figure 2B is a cross-sectional side view of the transducer of Figure 2A;

Figure 2C is a cross-sectional side view of the imaging device of Figure 2A disposed within a diagnostic or therapeutic assembly;

Figure 2D is a flow chart of a method of placing a tip of a diagnostic or therapeutic assembly according to an embodiment;

Figure 2E is a flow chart of a method of placing a tip of a diagnostic or therapeutic assembly according to another embodiment;

Figure 2F is a cross-sectional side view of an alternative embodiment of an imaging device;

Figure 2G is a perspective view of the imaging drill bit of Figure 2F;

Figure 3A is a cross-sectional side view of an imaging device according to another embodiment;

Figure 3B is a cross-sectional side view of the imaging device of Figure 3A disposed within a biopsy or therapeutic assembly;

Figure 4 is a cross-sectional side view of an imaging device according to another embodiment;

Figure 5 shows a cartooned example of an image that may be produced by an imaging device according to embodiments disclosed herein, including a forward-looking conical section of tissue displayed in a circular format;

Figure 6A is a cross-sectional side view of an imaging device according to another embodiment;

Figure 6B is an enlarged view of the scanning assembly of Figure 6A;

Figure 7A is a cross-sectional side view of an imaging device according to another embodiment;

Figure 7B is an enlarged view of the scanning assembly of Figure 7A;

Figure 8A is a cross-sectional side view of an imaging device according to another embodiment; '

Figure 8B is an enlarged view of the scanning assembly of Figure 8A;

Figure 9A is a cross-sectional side view of an imaging device according to another embodiment;

Figure 9B is a perspective view of the sector scanning mechanism of Figure 9A;

Figure 9C is a cross-sectional side view of an imaging device according to another embodiment;

Figure 9D is a perspective view of the sector scanning mechanism of Figure 9A;

Figure 10A is a cross-sectional perspective side view of an imaging device according to another embodiment;

Figure 10B is a perspective view of the sector scanning mechanism of Figure 10A;

Figure 10C is a cross-sectional perspective view of an imaging device according to another embodiment;

Figures 11A-11C are perspective views of scanning assemblies according to embodiments;

Figure 12 is a cross-sectional side view of an imaging device according to another embodiment; and

Figures 13A-13C are flowcharts of methods of imaging according to embodiments.

### DETAILED DESCRIPTION

Many medical diagnoses and therapies are performed with the aid of a needle. In some cases cells or fluids are removed from the suspect tissue for examination ex- vivo to determine the state of the tissue or contents of the cells or fluids. Once the diagnosis has been made, needles may be use to deliver therapies in a minimally invasive fashion. These therapies may involve, for example, Radio Frequency (RF) ablation, chemical ablation, laser ablation, vessel cautery, radioactive seed implants, cryotherapy, and photodynamic therapy. Frequently the accuracy with which a needle may be placed in the tissue to be examined or treated is insufficient, thereby requiring more invasive surgical approaches. To assist in the placement of these needles, image-guided methods have been developed using various medical imaging modalities. These imaging modalities are typically standard imaging systems that have been adapted to show the path of the needle as it is advanced to the tissue of interest In some cases, however, a more precise method of guiding a needle is beneficial. This is particularly true when the size of the tissue to be examined or treated is small compared to the resolution of the standard imaging system. It may also be true when the position of the tissue is significantly displaced by the advance of the needle toward it. Lesions smaller than, for example, ∼ 3 mm in size may be difficult to visualize with external image guidance systems.

Breast cancer is a major health problem for women. Early detection and treatment of tumors is crucial to the long-term survival of patients. As such, women are encouraged to perform regular self-examinations and receive an annual breast scan after the age of forty. When suspicious lesions are found, they are typically subjected to needle biopsy to determine the nature of the cells that form the lesion. This is done by inserting a large diameter needle, typically under image guidance, into the suspect tissue whereby a small sample is suctioned into the tip of the needle, removed from the body, and examined under microscope by a cytopathologist. This biopsy is often performed with external ultrasonic or mammographic image guidance of the trajectory of the needle from the skin surface of the breast to the lesion. It is important to verify the position of the needle tip relative to the suspect lesion before acquiring the tissue sample for further evaluation. A failure to do so may result in adjacent normal tissue being mistaken for the suspect tissue. On occasion the advance of the biopsy needle may displace the suspect tissue from its original position thereby making the collection of the desired tissue difficult or impossible.

Another problem with this approach is that not all lesions visible on x-ray mammography can also be seen using external ultrasound. In addition, it is also very difficult to obtain image guidance using either external ultrasound or mammography for lesions that are in, for example, the axilla or very posterior lesions near the patient's chest wall.

Image guidance also helps physicians to position needles, and similar devices such as reamers and drills, for other medical procedures, such as drainage, precision injections, pedicle screw placements, amniocentesis, cordocentesis, brachytherapy seed implantations, and transabdominal chorionic villus sampling. In all of these cases, it may be difficult to maintain the trajectory of the needle within the image plane or field of view of the external imaging system. In cases where very precise needle positioning is required in soft tissues, the resolution of the external imaging system may be inadequate to ensure that the needle, reamer or drill tip is in the desired location.

Embodiments relate generally to the fields of diagnostic and therapeutic medicine, and more specifically, to an imaging device which may include may be deployed within a needle or other diagnosis therapy device to aid in guiding the needle or other diagnosis therapy device to a precise location for diagnoses and treatment of, for example, soft tissue lesions, and methods of imaging using such an imaging device. At least one embodiment described herein incorporates a very low cost imaging system and an inexpensive imaging device design that may be sold as a disposable device. While an external imaging system, or previously acquired external images, may be used to position a tip of the imaging device in the vicinity of suspect tissue, an operator of the imaging device according to embodiments may switch to an integrated imaging device and tip imaging system, and more precisely direct the tip of the imaging device to the desired location when a lesion is very small. The combined imaging system also takes up little space and may be integrated into the bed or procedure table that a physician would normally use.

All of the embodiments disclosed herein allow for a more accurate placement of a tip of a needle, reamer or drill assembly by virtue of imaging tissue structures that lie distal to the tip and advancing accordingly to a desired location. The imaging device, imaging system, and methods of imaging according to embodiments allow diagnostic or therapeutic needle-based (reamer or drill based) procedures, for a more accurate sampling of the suspect tissue for later examination and in some cases, the direct treatment of a lesion using various therapies or placement of pedicle screws or other like devices.

Embodiments disclosed herein allow for the visualization of smaller lesions during diagnostic and therapeutic procedures, a more accurate sampling of suspect tissue for later examination and in some cases, direct treatment of a lesion using various therapies. The imaging device, imaging system, and methods of imaging may include an imaging device that may fit inside, for example, a diagnostic, biopsy, drainage, or therapeutic assembly. The imaging device may reside inside the diagnostic, biopsy, drainage, or therapeutic assembly as the combined device is advanced to a desired location. Images may be produced that allow for accurate placement of the diagnostic, biopsy, drainage, therapeutic assembly or pedicle drill. A transducer may be located near a distal tip of the imaging device at an angle relative to a central longitudinal axis (referred to hereinafter as the "the central axis") of the imaging device. The transducer may be housed in a scanning assembly that sweeps the transducer through an angle of acoustic scan. When the imaging device is rotated, the scanning assembly may transmit and collect echoes from a forward- looking sector or conical section of tissue that lies just distal to a distal most tip of the imaging device. An image may be formed when the imaging device is rotated. As a sector angle may be swept out, the image in the corresponding sector of the display may be updated. The imaging device may be rotated through, for example, a full ∼360 degree sweep to display the entire conical section of tissue. Alternatively, the imaging device may be swept repeatedly back and forth over a narrower sector to transmit and receive the scan lines and update a display of an imaging system repeatedly only over the narrow sector. Using the images generated by the imaging device, the operator may manually maneuver the tip of the imaging device to a precise location that appears in the display. A second assembly for diagnosis or therapy may be preloaded onto the imaging device and when the imaging device is in place the second assembly may be advanced over the imaging device in a co-axial fashion to the desired location. Once the diagnostic or therapy assembly is in place, the imaging device may be withdrawn and the standard procedure may continue.

In another embodiment, both a forward-looking conical section of tissue and a forward-looking angular sector of tissue may be interrogated. In this case, the sector image may be made by "wobbling" the scanning assembly back-and-forth through a desired sector angle. This wobbling action varies the angle of the transducer with respect to a central axis of the imaging device. The wobbling may be accomplished through a variety of mechanical mechanisms that allow a force to be exerted on the scanning assembly while a second angle encoder is recording a position of the proximal portion of the mechanism.

If the operator desires to change the forward-looking cone angle, he or she may do so by adjusting and setting the transducer angle with respect to the central axis of the imaging device. Further, if the operator wishes to interrogate, for example, an entire three- dimensional forward-looking conical volume this may also be done by sweeping out a series of sectors as the imaging device is rotated in the tissue about the central axis of the- imaging device. Analogously, this may be achieved by rotating the scanning assembly though a series of cones while varying the angle from a maximum possible angle with respect to the central axis of the imaging device to in-line with the central axis of the imaging device. Software may be provided to keep track of both the rotational angle of the housing and the angle of the transducer about the central longitudinal axis of the scanning assembly, and appropriately recording these along with the echo data that returns to the transducer in each position.

As set forth above, the imaging device according to certain embodiments disclosed herein may be used with or incorporated into a diagnostic or therapeutic assembly. For example, a standard diagnostic or therapeutic assembly may be advanced or retracted over the imaging device co-axially. Such a combination allows the diagnostic or therapeutic assembly to be accurately directed to an area of interest based on information in the images produced using signals from the imaging device. For example, the imaging device may fit inside a diagnostic or therapeutic assembly, such as a biopsy, drainage, or other type therapy assembly. The imaging device may reside inside the diagnostic or therapy assembly as the combined device is advanced to a desired location. Two-dimensional or three-dimensional images using signals from the imaging device may be produced that allow for the accurate placement of the diagnostic or therapy assembly.

In an alternative embodiment, the imaging device may take the form of an imaging drill or imaging reamer that may be used, for example, to create precisely located pilot holes in the pedicles of the vertebrae for spinal fixation procedures. In such an embodiment, a fluted drill-like surface or a ribbed reamer surface may be substituted for the smooth surface of the imaging device.

Further, the imaging device according to certain embodiments disclosed herein may be manually rotated or electronically rotated. Also, the imaging device according to certain embodiments disclosed herein may be rotated ∼360° to produce an image, for example, a forward-looking circular or conical image. The forward-looking circular or conical image may be representative of tissue on a surface of a cone. Alternatively, the scanning assembly according to certain embodiments disclosed herein may be rotated about the central longitudinal axis of the scanning assembly to scan a sector image, for example, a forward-looking sector image. Additionally, the scanning assembly according to certain embodiments may be rotated to produce a three-dimensional volumetric image by rotating a scanned sector image.

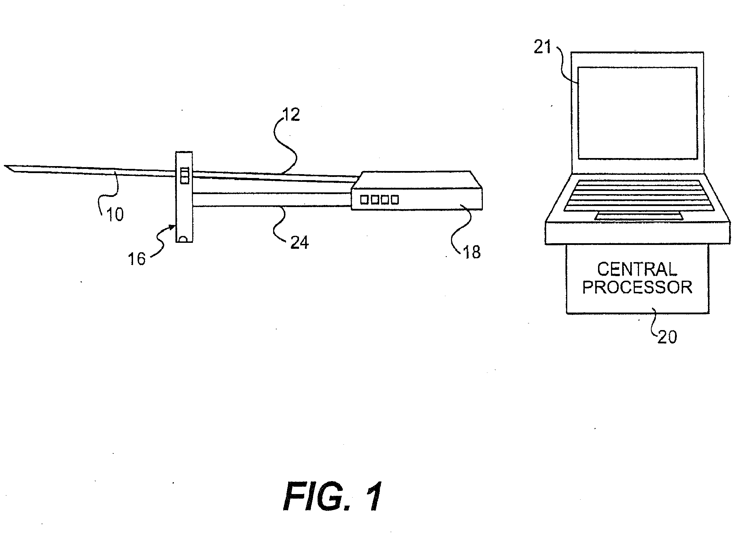

In the following discussion, the imaging device according to embodiments is disclosed as utilized with an imaging system shown in Figure 1 and described in US Patent Application No. 11/053,141, which is hereby incorporated by reference. However, it should be understood that the imaging device according to embodiments may be utilized with other systems as well.

As set forth above, an imaging system and an imaging device according to embodiments are disclosed herein. As discussed above, Figure 1 shows an imaging system described in US Patent Application No. 11/053,141, which is hereby incorporated by reference. This system is a low-cost ultrasonic imaging system. Unlike conventional mechanically steered imaging systems, this system does not require a motor to rotate or wobble the scanning assembly. Instead, driving the scanning assembly may be done manually by the operator and the image displayed as the transducer is swept through a section of tissue to be imaged. In this system, the imaging device 10 may emit a transmit signal and receive echoes, which may be carried to an electronics module 18 by a cable 12. An angle of rotation of the imaging device 10 may be encoded by an angle encoder 16 and the quadrature signals may be carried from, and power may be carried to the angle encoder 16 via a small multiconductor cable 24.

Inside the electronics module 18 the echoes may be demodulated and passed to a Central Processor 20 for display on a monitor 21. In the Central Processor 20, the demodulated echo data may be combined with the image assembly angle information to place the echo line, representing the tissue being imaged, in the correct geometric location as described in copending U.S. Patent Application No. 11/437,687, filed May 22, 2006, entitled "Apparatus and Method for Rendering for Display Forward-Looking Image Data" (Attorney Docket No. NOVS-0004), which is hereby incorporate by reference. Finally, the image may be rendered for display on the monitor 21.

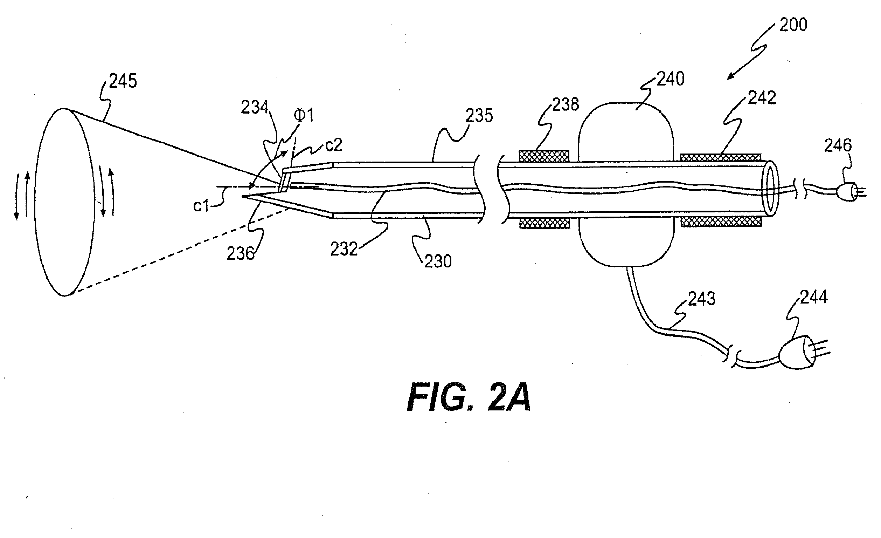

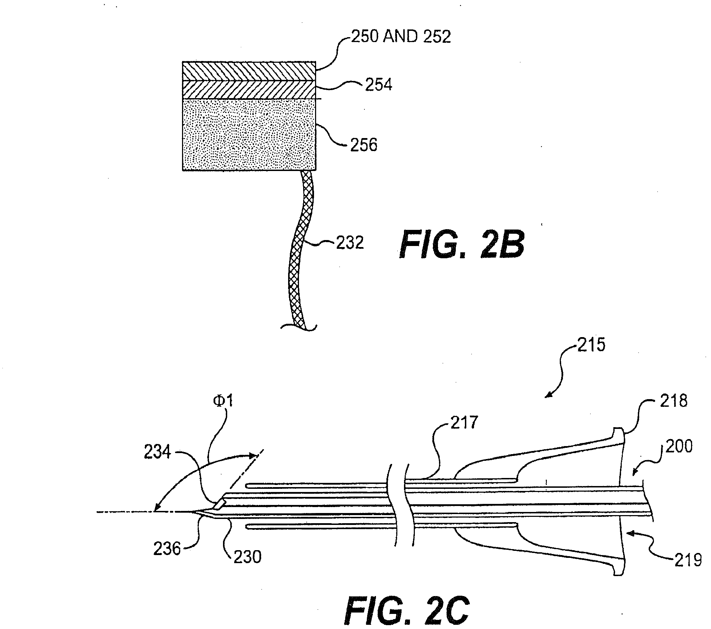

Figure 2A is a side view of an imaging device according to an embodiment. Figure 2B is a cross-sectional side view of the transducer of Figure 2A. Figure 2C is a cross- sectional side view of the imaging device of Figure 2A disposed within a diagnostic or therapeutic assembly.

The imaging device 200 of Figure 2A maybe in the form of an imaging needle assembly, and may be designed to form a conical forward-looking image 245, as shown in Figure 2A. The imaging device 200 may include a housing 230- An angle encoder 240 may be provided to encode a rotational angle of the housing 230 relative to the rest of the device and a hand grip. The grip, which may be in the form of knurled sleeves 238, 242, may be provided to assist an operator in grasping the housing 230. A transducer 234 may be positioned on a distal tip 236 of the imaging device 200. The transducer 234 may include, for example, an ultrasonic transducer, such as the transducer shown in Figure 2B. The transducer 234 may be oriented along a line C2 at an angle Φ1 from a central axis CI of the imaging device 200 between ∼0 degrees and ∼90 degrees. For the purpose of illustration, an angle in the range of ∼10 degrees and ∼ 30 degrees is shown. The transducer 234 may communicate through a coaxial cable 232 and a connector 246 to an imaging system, such as the imaging system as shown in Figure 1.

As shown in Figure 2B, the transducer may include, for example, a face plate 250 that serves as a matching layer 252, a piezoelectric transducer 254, and an absorptive backing layer 256 that attenuates the sound waves emanating for a rear side of the piezoelectric transducer 254. The matching layer 252 may be made of an appropriate material such that it is, for example, an approximately ^{∧}A wavelength thick and has an acoustic impedance that is the geometric mean of the piezoelectric material and the body tissue. It may also be made of multiple layers of appropriately selected materials so as to broaden a bandwidth of the transmitted and received sound waves. A frequency of the transducer may be selected to provide sufficient resolution to make adequate images of small structures, for example, < ∼3 mm an operator wishes to locate and yet allow for sufficient penetration so the operator may visualize a larger landscape that aids in locating more distant lesions. An aperture (or size and shape) of the transducer may be configured to fit within the imaging device 200 and may be used primarily as an unfocused near- field imaging device, although it may include a lens for focusing, if desired.

The angle encoder 240 may be formed integral with the imaging device 200. The angle encoder 240 may include a cable 243 and a connector 244.

In operation, an operator may rotate the housing 230, for example, by grasping the knurled sleeves 238 or 242 between the thumb and index finger. The integral angle encoder 240 may encode the rotational angle in real time as the housing 230 is rotated.
Echo information may be carried back to an electronics module 18 of an imaging system, such as the imaging system shown in Figure 1, by the coaxial cable 232 and the connector 246. Angle information may be carried back to the electronics module 18 via the cable 243 through the connector 244. As the operator sweeps out an angular sector by rotating the housing 230, the image may be updated in real time on the display module 21.

Figure 2C is a cross-sectional side view of the imaging device of Figure 2A disposed within a diagnostic or therapeutic assembly. The imaging device may be in the form of an imaging needle assembly and the diagnostic or therapeutic assembly in the form of a diagnostic or therapeutic needle assembly. The distal portion 235 of the imaging device 200 from the knurled sleeve 238 on may fit inside of an exterior housing 217 of the diagnostic or therapeutic assembly 215, as shown in Figure 2C. When a desired location of the distal tip 236 of the imaging device 200 has been reached, the imaging device 200 may be removed from a lumen 219 of the diagnostic or therapeutic assembly 215 and the standard procedure may continue. The diagnostic or therapeutic assembly 215 may further include a connector 218, such as a leur connector.

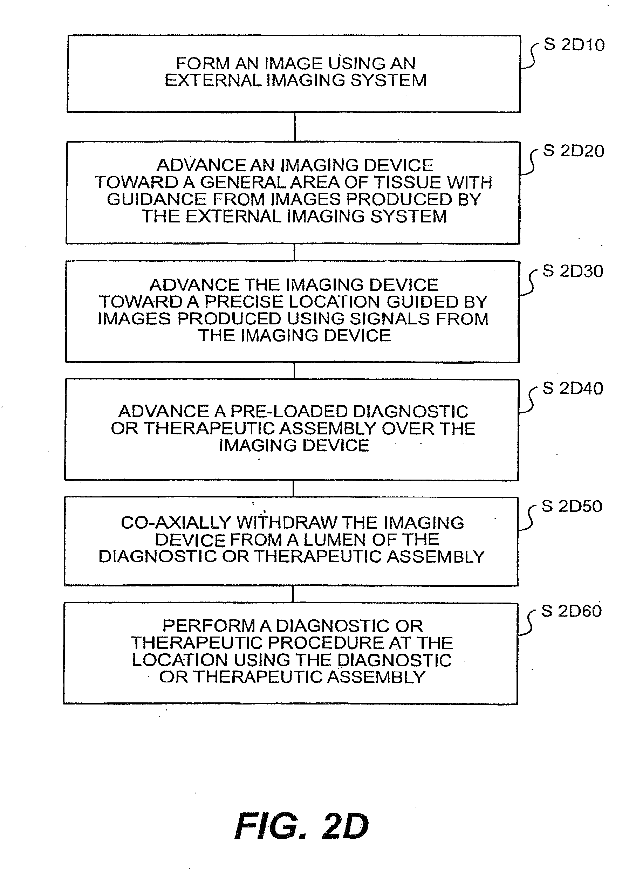

Figure 2D is a flow chart of a method of placing a tip of a diagnostic or therapeutic assembly at a specific location in tissue according to an embodiment. The imaging device may be in the form of an imaging needle assembly and the diagnostic or therapeutic assembly in the form of a diagnostic or therapeutic needle assembly. The method may include forming an image using an external imaging system, such as a commercially available ultrasonic imaging system (step 2D10). An imaging device may then be advanced toward a general area of tissue with guidance from images produced by the external imaging system (step 2D20). Next, the imaging device may be advanced to a precise location guided by images produced using signals from the imaging device (step 2D30). Thereafter, a pre-loaded diagnostic or therapeutic assembly may be advanced co-axially over the imaging device (step 2D40), and the imaging device co-axially withdrawn from a lumen of the diagnostic or therapeutic assembly (step 2D50). A diagnostic or therapeutic procedure may then be performed at the location using the diagnostic or therapeutic assembly (step 2D60). This method may be employed in, for example, general biopsy procedures, breast biopsy procedures, prostate biopsy procedures, aspiration procedures, amniocentesis procedures, cordocentesis procedures, and transabdominal chorionic villus sampling procedures. Further, the method may be employed to perform a therapeutic procedure, such as RF ablation, a chemical injection, and a brachytherapy seed placement procedure. With this method, a lesion to be diagnosed or treated may be less than ∼3 millimeters in size.

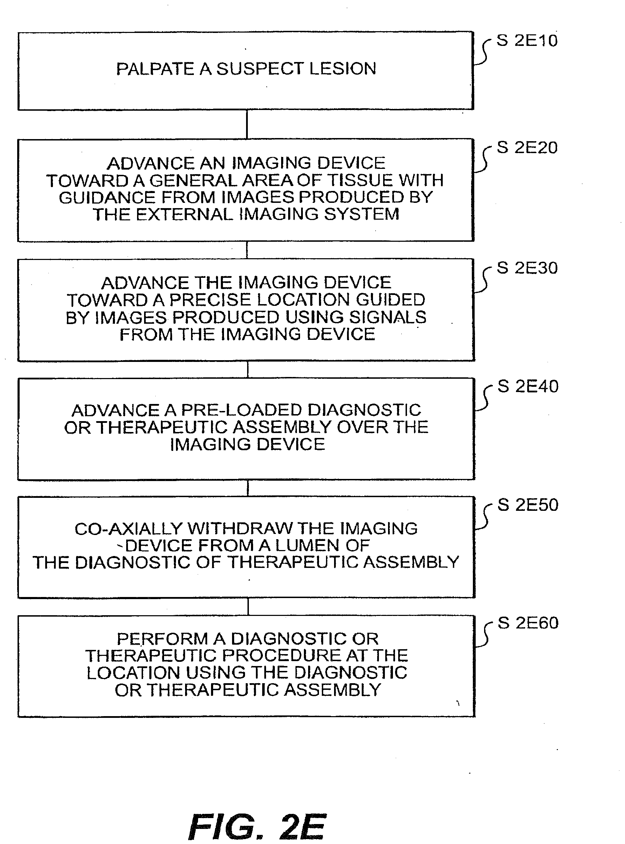

Figure 2E is a flow chart of a method of placing a tip of a diagnostic or therapeutic assembly at a specific location in tissue according to an embodiment. The imaging device may be in the form of an imaging needle assembly and the diagnostic or therapeutic assembly in the form of a diagnostic or therapeutic needle assembly. The method may include palpating a suspect lesion (step 2E1 0). An imaging device may then be advanced toward a general area of tissue with guidance from images produced by the external imaging system (step 2E20). Next, the imaging device may be advanced to a precise location guided by images produced using signals from the imaging device (step 2E30). Thereafter, a pre-loaded diagnostic or therapeutic assembly may be advanced co-axially over the imaging device (step 2E40), and the imaging device may be co-axially -withdrawn from a lumen of the diagnostic or therapeutic assembly (step 2E50). Then, a diagnostic or therapeutic procedure may be performed at the location using the diagnostic or therapeutic assembly (step 2E60). This method may be employed in, for example, general biopsy procedures, breast biopsy procedures, prostate biopsy procedures, aspiration procedures, amniocentesis procedures, cordocentesis procedures, and transabdominal chorionic villus sampling procedures. Further, the method may be employed to perform a therapeutic procedure, such as RF ablation, a chemical injection, and a brachytherapy seed placement procedure. With this method, a lesion to be diagnosed or treated may be less than ∼3 millimeters in size.

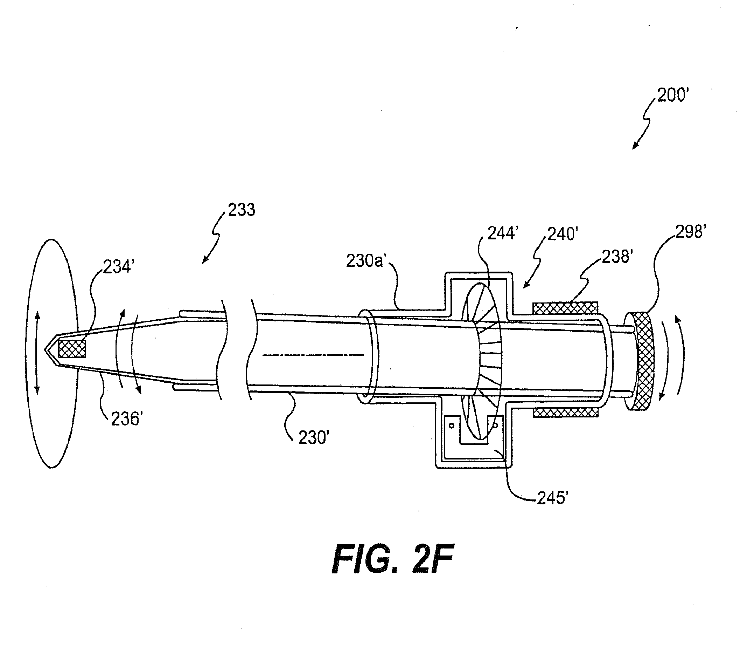

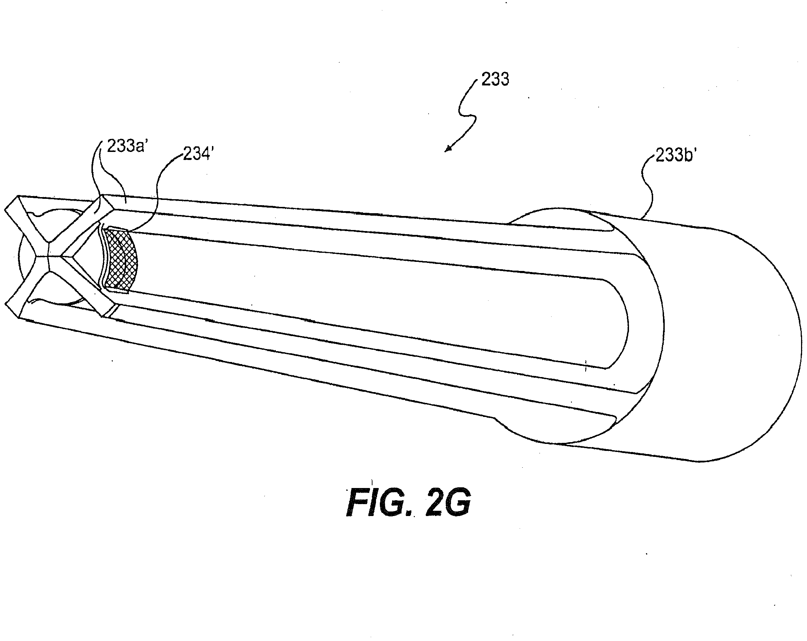

As set forth above, in alternative embodiments, the imaging device may take the form of an imaging drill or imaging reamer that may be used, for example, to create precisely located pilot holes in the pedicles of the vertebrae for spinal fixation procedures. In such embodiments, a fluted drill-like surface or a ribbed reamer surface may be substituted for the smooth surface of the imaging device. Examples of such alternative embodiments are shown in Figures 2F and 2G. That is, Figures 2F shows an imaging drill bit 233 provided as a distal end 236' of the imaging device 200'. The imaging drill bit 233 and housing 230' may be rotated by rotating knob or sleeve 298'. An angle encoder 240', including photo interrupter 245* and slit wheel 244', may be provided housed within outer sleeve 230a A grip, which may be in the form of knurled knob 238', may be provided on the outer sleeve 230a to allow either rotating or oscillating motion. Rotation creates the circular image 245' depicted.

Figure 2G shows the imaging drill bit 233' depicted with the transducer 234" and cutting edges 233a' along both sides of four flutes along a tapered body connected to straight shank 233b'. The straight shank 233b 'may mount permanently in the imaging device 200'.

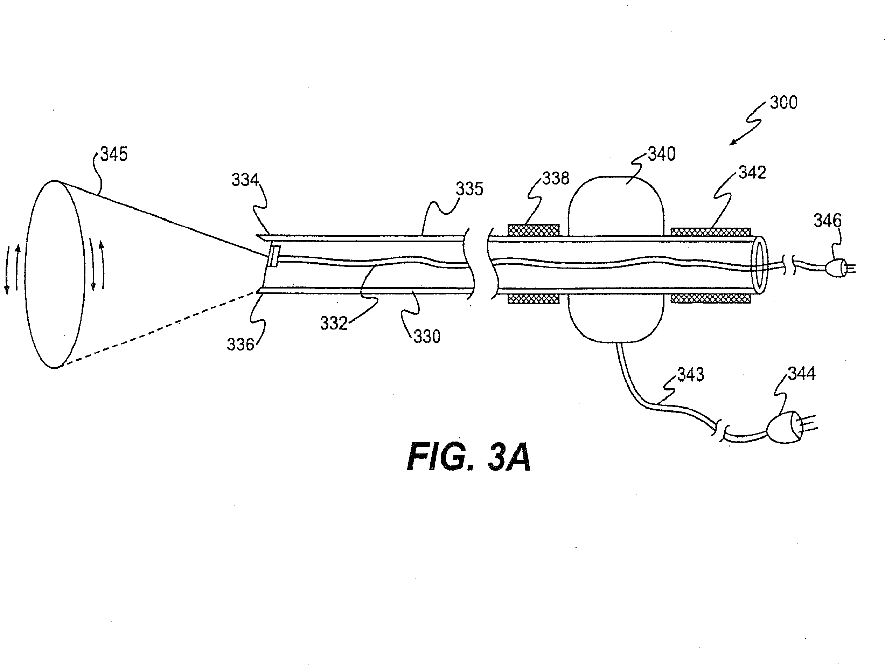

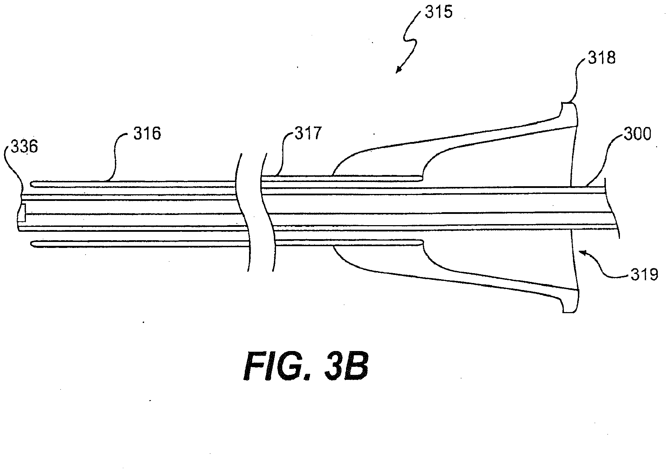

Figure 3A shows another embodiment of an imaging device. Figure 3B is a cross-sectional side view of the imaging device of Figure 3A disposed within a biopsy or therapeutic assembly. The imaging device may be in the form of an imaging needle assembly and the diagnostic or therapeutic assembly in the form of a diagnostic or therapeutic needle assembly. In Figures 3A-3B, like reference numerals have been used to indicate like elements to the embodiment of Figures 2A-2B, and repetitive disclosure has been omitted.

The imaging device 300 of Figures 3A-3B may be in the form of an imaging needle assembly. In the embodiment of Figures 3A-3B, a distal end 236 of the imaging device 300 may be substantially blunt rather than sharp. Further, the imaging device 300 may fit completely inside a biopsy or therapeutic assembly 315, as shown in Figure 3B, and provide images of tissue at a distal-most tip of the biopsy or therapeutic assembly 315. An imaging device 300 such as that shown in Figure 3A is particularly useful in aiding precise placement of a second tip 316, as shown in Figure 3B, that may be used for, for example, a drainage procedure, chemical injection, amniocentesis, cordocentesis, or transabdominal chorionic villus sampling. As the biopsy or therapeutic assembly 315 is advanced toward a target tissue, images may be obtained and mid-course corrections may be made to ensure that the tip of the biopsy or therapeutic assembly 315 may be precisely located in, for example, the desired tissue, vessel, lumen, or any other anatomical structure. The biopsy or therapeutic assembly 315 may further include a connector 318, such as a leur connector.

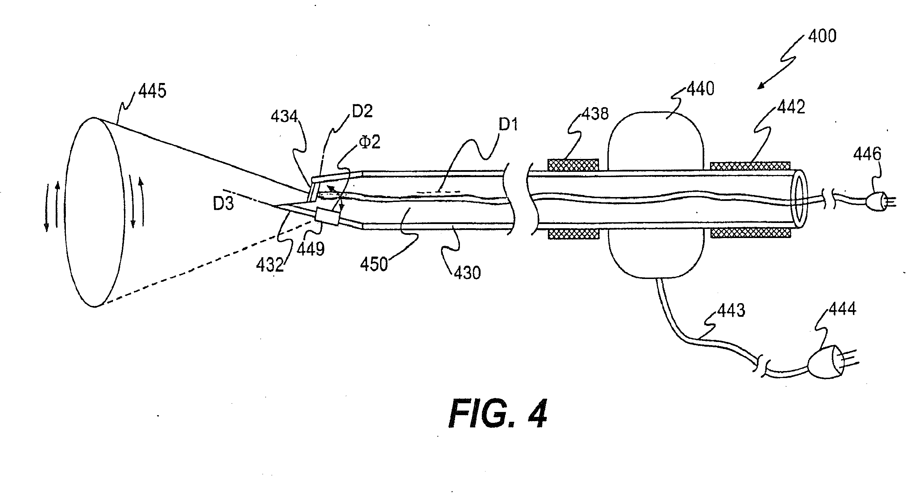

Figure 4 shows another embodiment of an imaging device. In Figure 4, like reference numerals have been used to indicate like elements to the embodiments of Figures 2A-3B, and repetitive disclosure has been omitted.

The imaging device 400 of Figure 4 may be in the form of an imaging needle assembly. In the embodiment of Figure 4, a treatment device, shown in this embodiment as an ablation device 449, such as a radiofrequency (RF) ablation antenna, may be incorporated into the distal end 436 of the imaging device 400 so that tissue in a target location may be ablated or cauterized. A central axis of the imaging device 400 is designated by reference numeral DI in Figure 4. The ablation device 449 may be located along a line D3, as shown in Figure 4, at an angle Φ2, shown in this embodiment∼180 degrees of rotation around the central axis of the imaging device from a line D2 on which the transducer 434 is positioned. The location of the ablation device 449 may be displayed on images that are created to allow the operator to image, then ablate or cauterize specific areas of tissue that appear in the image, such as a conical forward-looking image. Ablation energy may be carried to the ablation device 449 via cable 450 and attached to the electronics module 18 via a connector 446. The specific shape of the ablation device 449 may be designed to ablate or cauterize a variety of small volume shapes near the tip or distal end 436 of the imaging device 400. Likewise, the specific electrical signal sent to the ablation device 449 may be optimized for either ablation or cauterization.

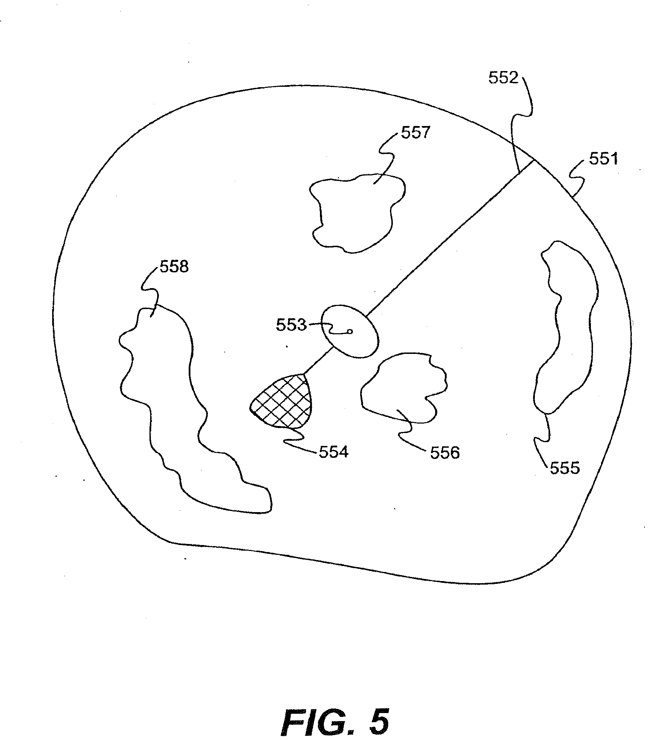

Figure 5 shows an example of how an imaging device according to embodiments may be displayed on a forward-looking guidance image. The image may be displayed as a two-dimensional image or as described in copending U.S. Patent Application No. 11/437,687, filed May 22, 2006, entitled "Apparatus and Method for Rendering for Display Forward-Looking Image Data" (Attorney Docket No. NOVS-0004), which is hereby incorporated by reference. As the imaging device is rotated, line 552, for example, of the image 551 displays a direction from which scan lines are being received. This corresponds to an angular orientation of the transducer. The location 554 where the therapy will occur may also be displayed and may be rotated with the transducer albeit, for example, ∼180 degrees away from where the new image lines are received. The center axis 553 of the imaging device may be stationary. Tissue structures 555, 556, 557, 558 will be seen if they intersect the forward-looking conical surface that is being swept out by the transducer.

As set forth above, the imaging device according to the embodiments of Figures 2A-4 are shown as forming a conical forward-looking image. However, a need may exist to scan in a sector mode, in addition to a circular or conical mode. The following embodiments accomplish this via a sector scanning mechanism that wobbles or oscillates a scanning assembly in order to scan a sector image.

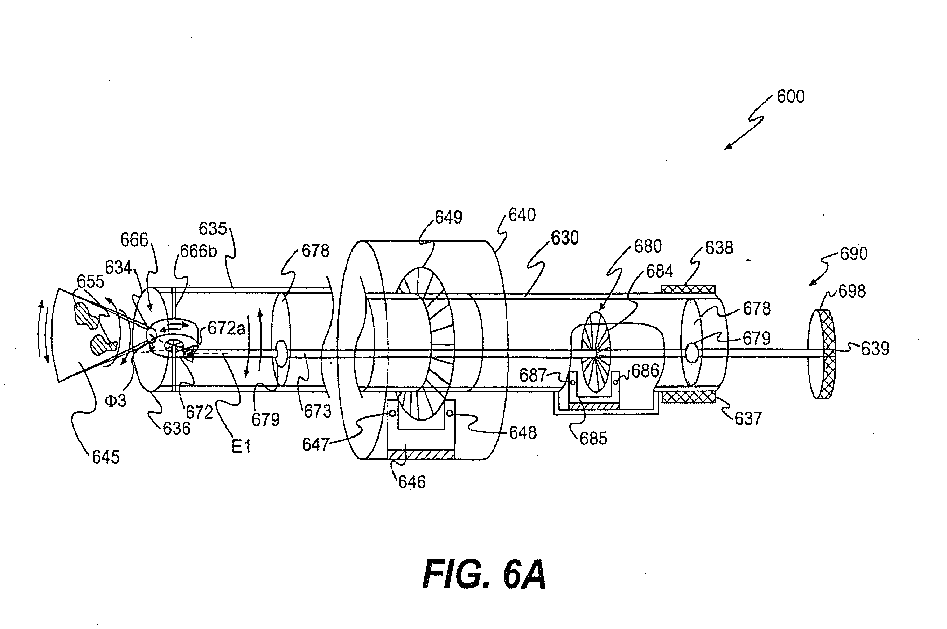

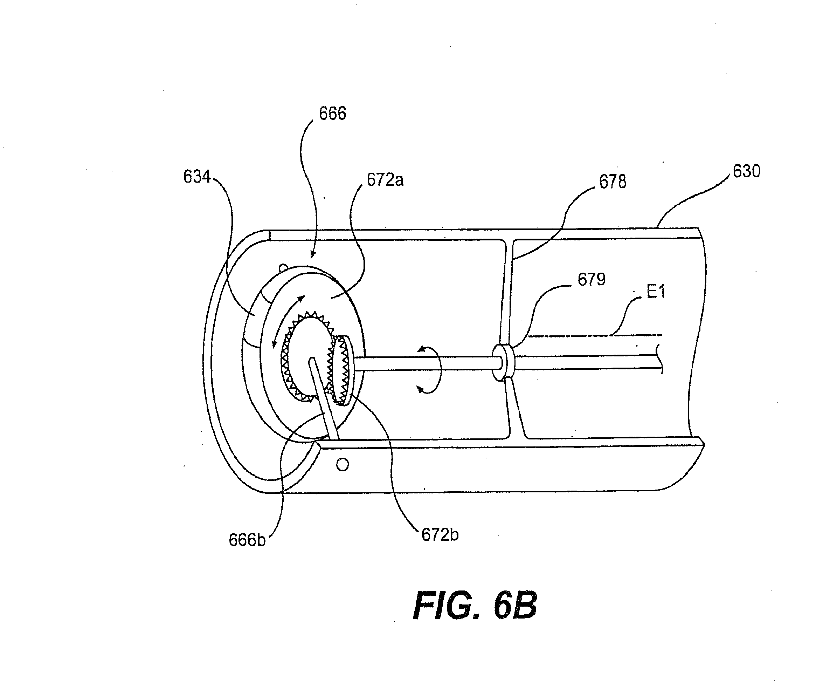

Figure 6A shows another embodiment of an imaging device. Figure 6B is an enlarged view of the scanning assembly of Figure 6A. In Figures 6A-6B, like reference numerals have been used to indicate like elements to the embodiments of Figures 2A-4, and repetitive disclosure has been omitted.

The imaging device 600 of Figures 6A-6B may be in the form of an imaging needle assembly. The embodiment of Figures 6A-6B includes a sector scanning mechanism 690 by which an angle Φ3 of a transducer 634a of the scanning assembly 666 from a central axis El of the imaging device 600 may be varied, thereby forming a sector image 645 from a distal end 636 of the imaging device 600. The embodiment shown in Figure 6A utilizes a drive shaft 673 that runs a length of the imaging device 600 and terminates at one end 636 with a gear 672b that drives the scanning assembly 666 (mounted on axle 666b) through a sector angle and at the other end 639 in a knob 698 after passing through a slotted encoder wheel 684 that allows the sector angle to be recorded.

This type of scanning is frequently referred to as mechanical "wobble" scanning. The sector image 645 that is produced may be manually swept out around central axis El by the operator when he or she rotates knurled sleeve 638 at the end 637 of the housing 630. The mechanism shown, in this embodiment, may incorporate a bevel gear 672a and bevel gear 672b that wobble the scanning assembly 666 on the axle 666b in response to the drive shaft 673 being oscillated back-and-forth by the turning of the knurled sleeve 638. The drive shaft 673 may be held in place by a support fixture 678 that captures a bearing 679, inside of which the drive shaft 673 rotates.

The drive shaft 673 may also be connected to an angle encoder 680. The angle encoder 680 may include a slit wheel 684 around which a photo interrupter 685 is positioned. The photo interrupter 685 may include a light emitting diode 687 and a photo detector 686 that sense each time a slit on the angle encoder slit wheel 684 passes between them. This may be similar to the angle encoding function performed by an angle encoder 640 for the imaging device 600 itself. The angle encoder 640 of the imaging device 600 may include a slit wheel 649 and a photo interrupter 646, which may include a light emitting diode 647 and a photo detector 648. However, the photo interrupter 685 may be attached to and rotate with the housing 630. The wiring of the two pho*o interrupters is not shown for clarity; however, it would be obvious to one skilled in the art.

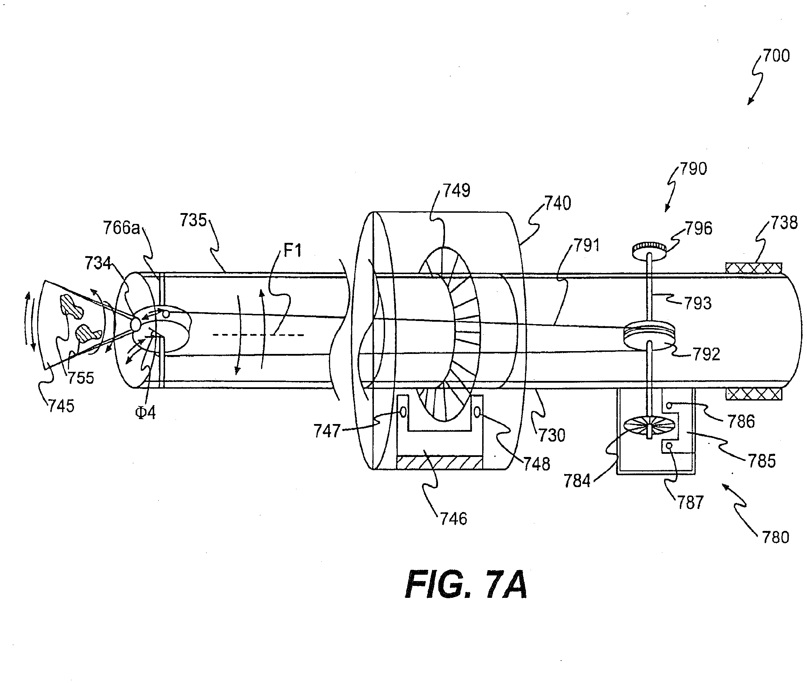

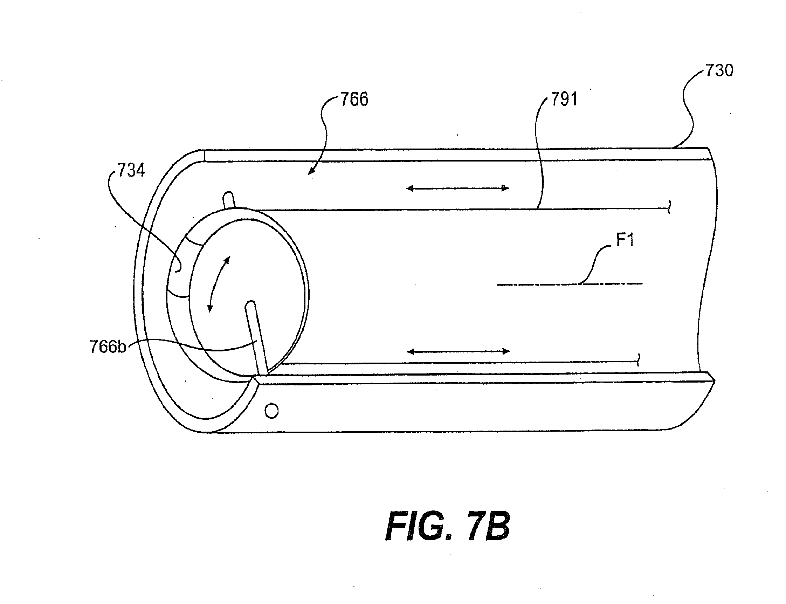

Figure 7A shows another embodiment of an imaging device. Figures 7B is an enlarged view of the scanning assembly of Figure 7A. In Figures 7A-7B, like reference numerals have been used to indicate like elements to the embodiments of Figures 2A-4 and 6A-6B, and repetitive disclosure has been omitted.

The imaging device 700 of Figures 7A-7B may be in the form of an imaging needle assembly. The embodiment of Figures 7A-7B may incorporate a sector scanning mechanism 790 to vary the angle of or wobble the scanning assembly 766. In Figure 7A, Fl represents a central longitudinal axis of the imaging device 700. The sector scanning mechanism may be in the form of a draw-string mechanism that allows the scanning assembly 766 mounted on axle 766b to be rotated an angle Φ4 with respect to the central axis of the imaging device 700. The draw-string mechanism 794 may include pull wires 791 that run a predetermined length along the imaging device 700 and wrap around a pulley 792 that communicates with an angle encoder 780. The angle encoder 780 may include a slotted encoder wheel 784 driven by the pulley 792 that allows an angle of the axle 793 to be recorded via photo interrupter 785. A knob 796 may be provided on one end of the axle 793 to rotate the axle 793.

In this embodiment, the pull wires or draw strings 791 may be used to drive the scanning assembly 766 back-and-forth through a desired sector angle to produce a sector image 745. The draw strings or pull wires 791 may wrap one or more times around the pulley 792, which in turn may be connected to the axle 793 that is rotated when the knob 796 is rotated. The encoder slit wheel 784 may be positioned on the other end of the axle 793 to trigger the photo interrupter 785, and thereby encode the angle of the pulley 792. The radius of the pulley 792 may be smaller or larger than the radius of the scanning assembly 766 to affect a gearing up or a gearing down.

The angle encoder 740 of the imaging device 700 may include a slit wheel 749 and a photo interrupter 746, which may include a light emitting diode 747 and a photo detector 748. However, the photo interrupter 785 may be attached to and rotate with the housing 730. The wiring of the two photo interrupters is not shown for clarity; however, it would be obvious to one skilled in the art.

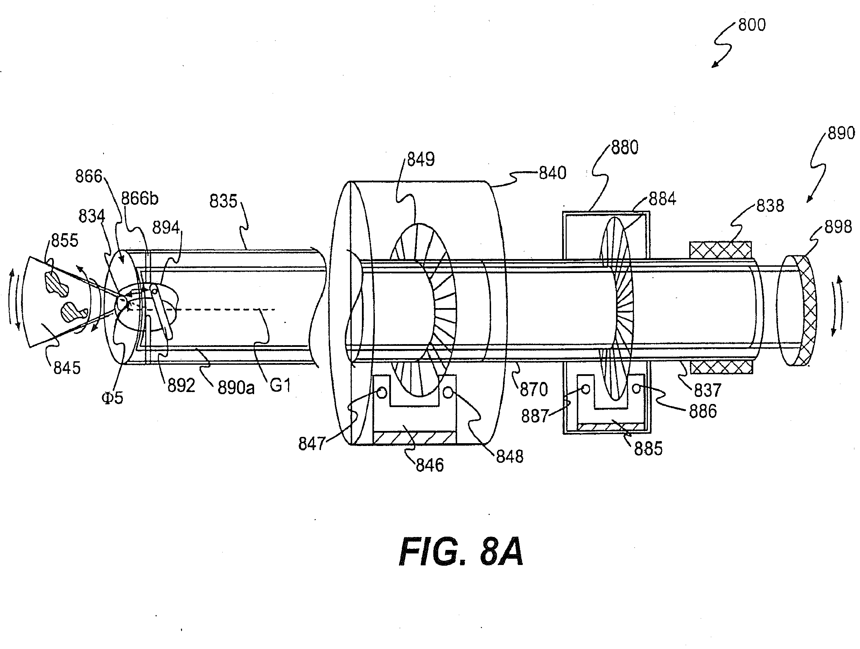

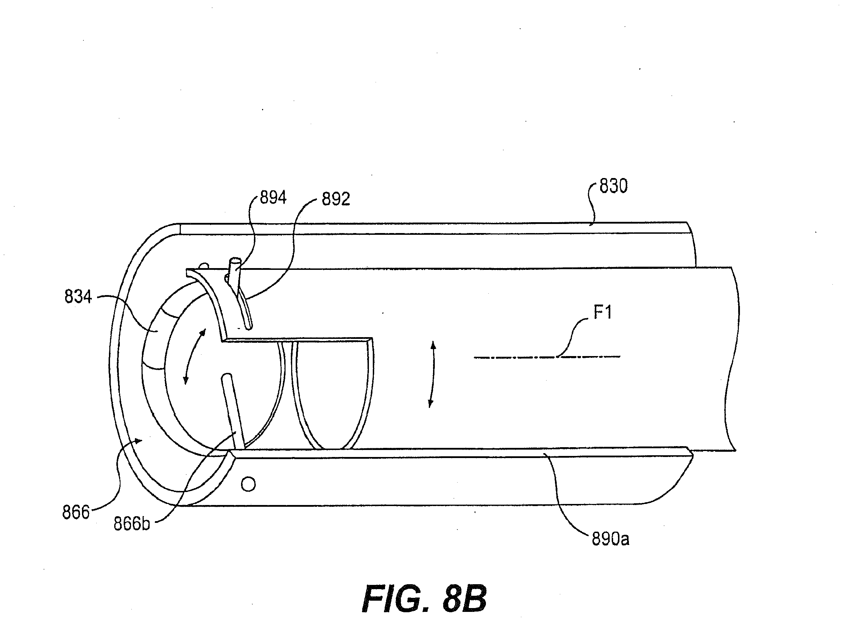

Figure 8A shows another embodiment of an imaging device. Figure 8B is an enlarged view of the scanning assembly of Figure 8A. In Figures 8A-8B, like reference numerals have been used to indicate like elements to the embodiment of Figures 2A-4 and 6A-7B, and repetitive disclosure has been omitted.

The imaging device 800 of Figures 8A-8B may be in the form of an imaging needle assembly. The embodiment of Figures 8A-8B may include a sector scanning mechanism 890 to vary the angle of or wobble the scanning assembly 866 to form a sector image 845. In Figures 8A-8B, Gl represents a central axis of the imaging device 800. The sector scanning mechanism 890 allows the scanning assembly 866 mounted on axle 866b to be rotated an angle Φ5 with respect to the central axis of the imaging device 800. This mechanism employs a concentric "drivetube" hypotube or inner tube 890a that has an angled slot 892 at a distal end that engages a pin 894 on the scanning assembly 866. An angle encoder 880, which may include a slotted encoder wheel 884, allows an angle of the inner tube 890a to be recorded. In this embodiment, the concentric "drivetube" hypotube drive or inner tube 890a on the inside of the housing 830 may be used as a drive shaft. At the distal end of the inner tube 890a, the angled slot 892 may be provided engaged by the pin 894 on the scanning assembly 866. When the tube 890a is rotated, the angled slot 892 pushes the pin 894 proximal and distal, thereby rotating the scanning assembly 866 back and forth over the sector angle to produce the sector image 845. The inner tube 890a may have a second oppositely angled slot on the other side (not shown) so as to engage a second pin (not shown) on the far side of the scanning assembly 866. The axle 866b (which corresponds to the central longitudinal axis of the scanning assembly) may, in fact, need to have an "omega" shape so as not to interfere with the inner tube 890a or the inner tube 890a may need to have part of its wall removed where the axle 866b enters the housing 830. This is not shown for simplicity. At the proximal end 837 of the housing 830, the inner tube 890a may have an encoder slit wheel 884 attached thereto which rotates with the inner tube 890a. As the encoder slit wheel 884 rotates, the photo interrupter 885 may generate pulses in response to each slit as it interrupts the light path from the light emitting diode 887 to the photo detector 886.

The angle encoder 840 of the imaging device 800 may include a slit wheel 849 and a photo interrupter 846, which may include a light emitting diode 847 and a photo detector 848. However, the photo interrupter 885 may be attached to and rotate with the housing 830. The wiring of the two photo interrupters is not shown for clarity; however, it would be obvious to one skilled in the art

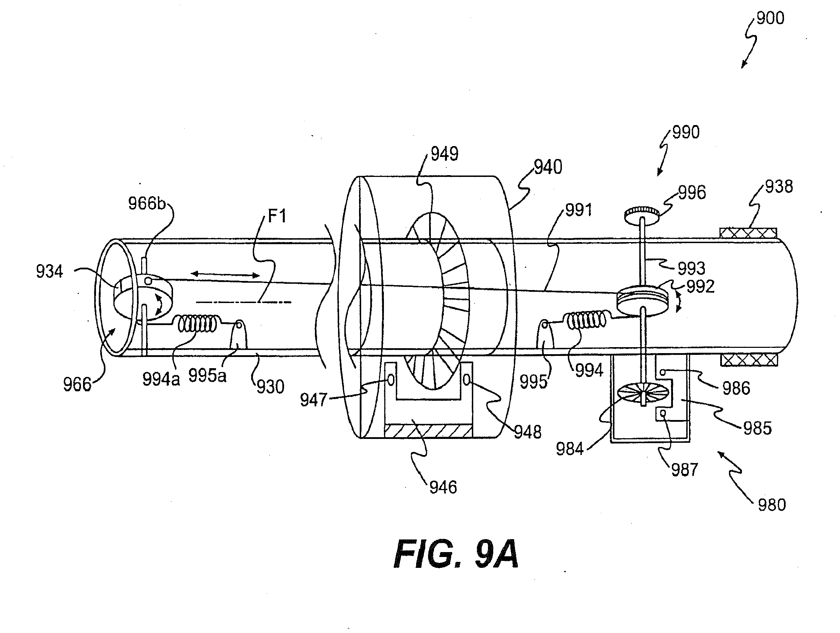

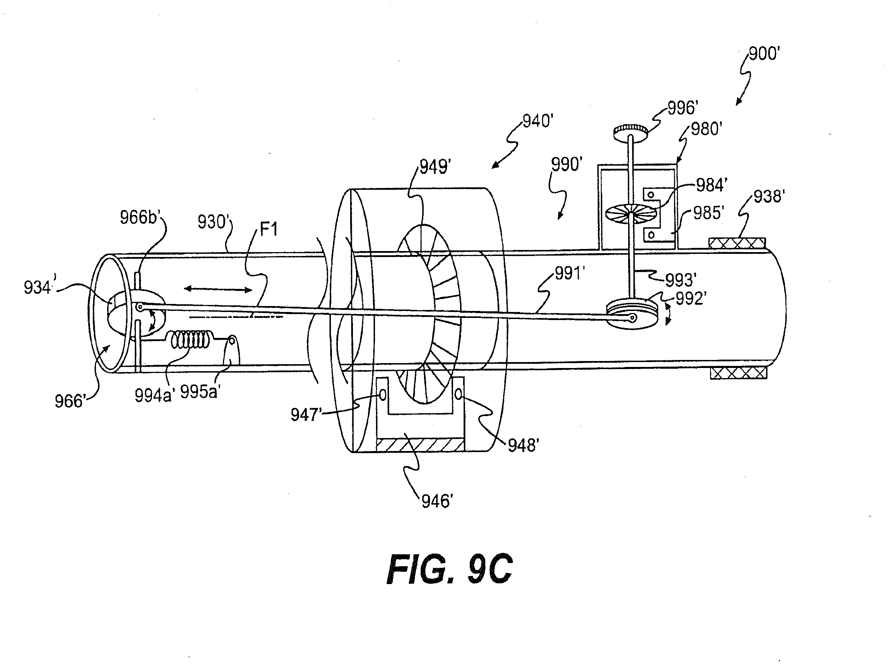

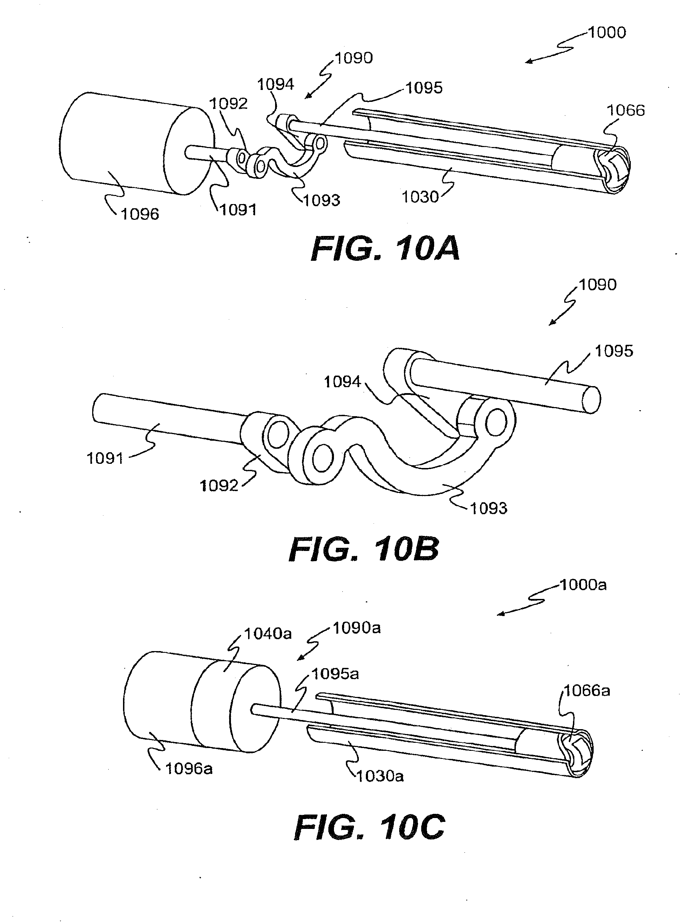

Figures 9A-10B show embodiments in which the scanning assembly may be oscillated by a sector scanning mechanism. The imaging device 900, 900, 1000, 1000a of each of Figures 9A-9B, 9C-9D, and 10A-10C may be in the form of an imaging needle assembly. Figures 9A-9B show an exemplary embodiment in which the sector scanning mechanism may include a pull and retract mechanism. Figures 9C-9D show an exemplary embodiment in which the sector scanning mechanism may include cranks and a connecting rod. Figures 10A-10B show an exemplary embodiment in which the sector scanning mechanism may include an oscillating drive shaft driven by an oscillating output mechanism. Figure 10C shows an exemplary embodiment in which the sector scanning mechanism may include an oscillating drive motor.

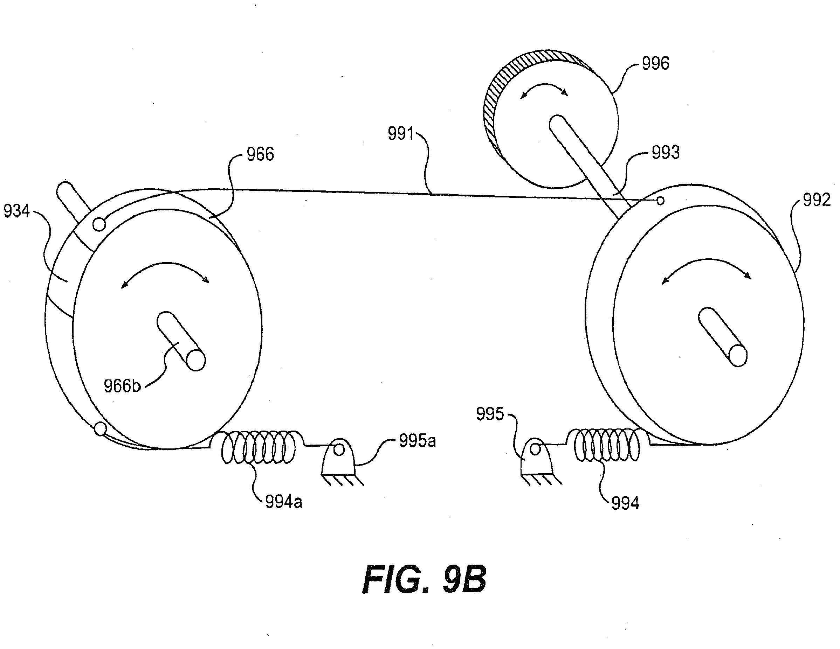

In the embodiment of Figures 9A and 9B, a sector scanning mechanism 990 may be provided in the form of a pull-and-retract mechanism. The pull-and-retract mechanism may include a cable 991 that pulls the scanning assembly 966, mounted on a pivot 966b through an angle via a pulley 992. The angularly displaced scanning assembly 966 may be returned to its original position by a spring 994, 994a pulling in the opposite direction. The spring 994, 994a may be connected to a base 995, 995a and to the scanning assembly 966 and/or the pulley 992, as shown in Figures 9A-9B.

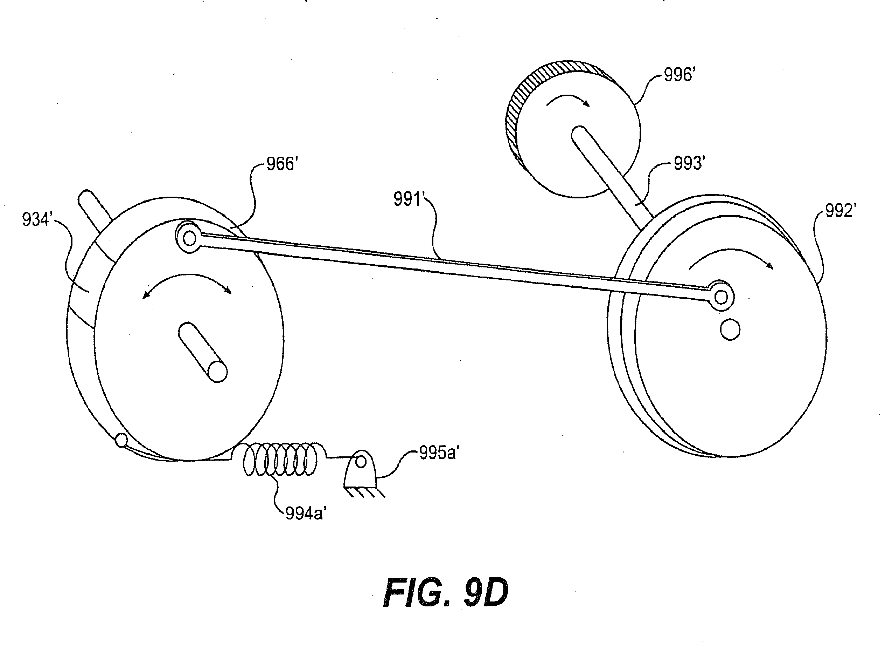

In the embodiment of Figures 9C and 9D, a sector scanning mechanism 990' may be provided in the form of a crank and rod mechanism that pulls and pushes the scanning assembly 966'. The crank and rod mechanism may include rod 991 ' connected to rotating crank 992' and to the scanning assembly 966', which may be mounted on pivot 966b'. The rotating crank 992 'may be mounted on shaft 993" and may be driven by gear 996'. A spring 994a' may be attached to base 995a' and scanning assembly 966' to prevent backlash.

The angle encoder 940 of the imaging device 900 may include a slit wheel 949 and a photo interrupter 946, which may include a light emitting diode 947 and a photo detector 948. However, the photo interrupter 985 may be attached to and rotate with the housing 930. The wiring of the two photo interrupters is not shown for clarity; however, it would be obvious to one skilled in the art.

In the embodiment of Figures 10A and 10B, a sector scanning mechanism 1090 may be provided in the form of an oscillating drive shaft 1095 driven by an oscillating output mechanism. That is, rotary motion from a motor 1096 and encoder 1040 may be converted to oscillating rotary motion by a small crank 1092 connected by a connecting rod 1093 connected to a larger crank 1094. The oscillating motion may be transmitted by an output or drive shaft 1095 to the scanning assembly 1066 where a pair of miter gears (not shown) transmit the oscillation to a shaft perpendicular to that of the drive shaft 1095. This right angle drive may be accomplished with miter, bevel, hypoid, helical, or brequet gears. In this way, the scanning assembly may be driven in a direction substantially perpendicular to that of the output or drive shaft 1095.

That is, as shown in Figures 10A, an imaging device 1000 according to this embodiment may include a scanning assembly 1066 disposed within housing 1030. The oscillating mechanism 1090 may be provided to convert rotary motion into an oscillating motion.

As shown in Figures 10A and 10B, the sector scanning mechanism 1090 may include an input shaft 1091, an input crank 1092, a connecting rod 1093, an output crank 1094, and an output shaft 1095. As the input shaft 1091 is rotated, the rotational motion may be converted to an oscillating motion by the input crank 1092, the connecting rod 1093, and the output crank 1094, so that the output shaft 1095 may be oscillated about its central longitudinal axis. The output shaft 1095 may be connected to the scanning assembly 1066 to rotate the transducer about an axis substantially perpendicular to the central axis of the output shaft 1095.

The sector scanning mechanism 1090a of Figure 10C may include drive or output shaft 1095a. An oscillating motor 1096a and encoder 1040a may oscillate the drive or output shaft 1095a, thereby oscillating the scanning assembly 1066a.

The imaging device of the embodiments of Figures 2A-4 and 6-10C are shown as forming a conical forward-looking image or a forward-looking sector image. However, the imaging device of the embodiments of Figures 2A-4 and 6-10C may be configured to produce other shaped images if so desired. Further, the imaging device of the embodiments of Figures 2A-4 and 6-10C may be mechanically or electronically operated. Additionally, although the imaging device of the embodiments of Figures 2A-4 and 6-10C are discussed as utilizing an ultrasonic transducer, the imaging device of the embodiments of Figures 2A-4 and 6-10C may utilize other types of transducers, such as an optical transducer, if desired.

Additionally, the embodiments of Figures 6A-8B and 9A-10C each include a sector scanning mechanism. One of ordinary skill in the art would recognize that these embodiments may be combined to produce desired scanning. Further, combining the sector scanning mechanism and a rotating mechanism configured to rotate the imaging device will allow the scanning, and thus imaging of volumes of tissues.

Referring to Figures 6A-6B, 7A-7B, 8A-8B, 9A-9B, 9C-9D, and 10A-10C, the operator may rotate the imaging device 600, 700, 800, 900, 900', 1000, 1000a to form an image of the tissue on a surface of a forward-looking conical surface or back and forth to form a forward-looking sector image 645, 745, 845, 948, 1048 of the tissue. If the operator desires, he/she can perform a series of scanning assembly rotations and sector angle wobbles to manually interrogate an entire three-dimensional volume of tissue in front of the imaging device 600, 700, 800, 900, 900', 1000, 1000a. This resultant three-dimensional echo data may then be displayed by techniques common in the field to show all of the tissue in a conical volume distal to the tip of the imaging device 600, 700, 800, 900, 900', 1000, 1000a.

That is, in addition to scanning a slice of anatomy, it may be advantageous for certain applications, for example, certain image reconstruction techniques, to scan volumes. Volumes may be rapidly scanned by combining, for example, a rotary oscillation with a sector scan to produce a volume scan. To accomplish this, the mounting containing the sector scan mechanism may be made to oscillate or rotate about a central axis of the imaging device. The scanning assembly may be inclined at an angle where a midpoint of the sector is at an angel to the central axis of the imaging device that is half the total sector angle to most efficiently use the available scanning time. This avoids "wasting time" repeatedly scanning previously scanned tissue.

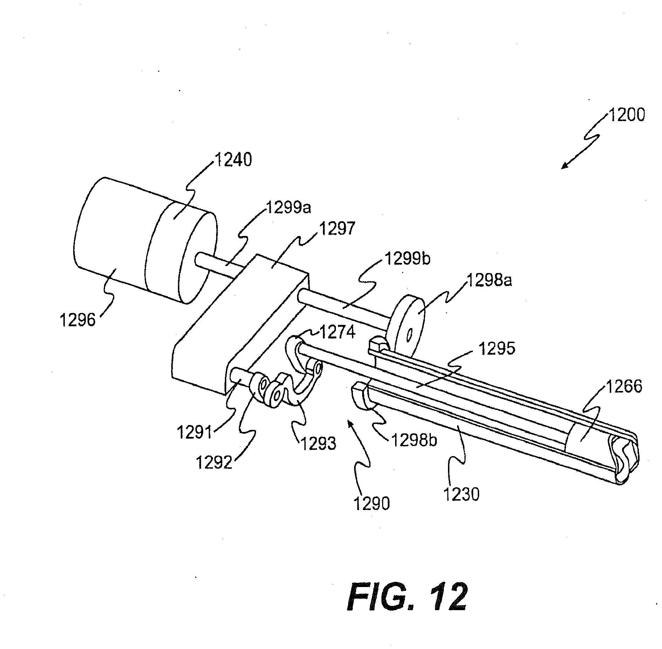

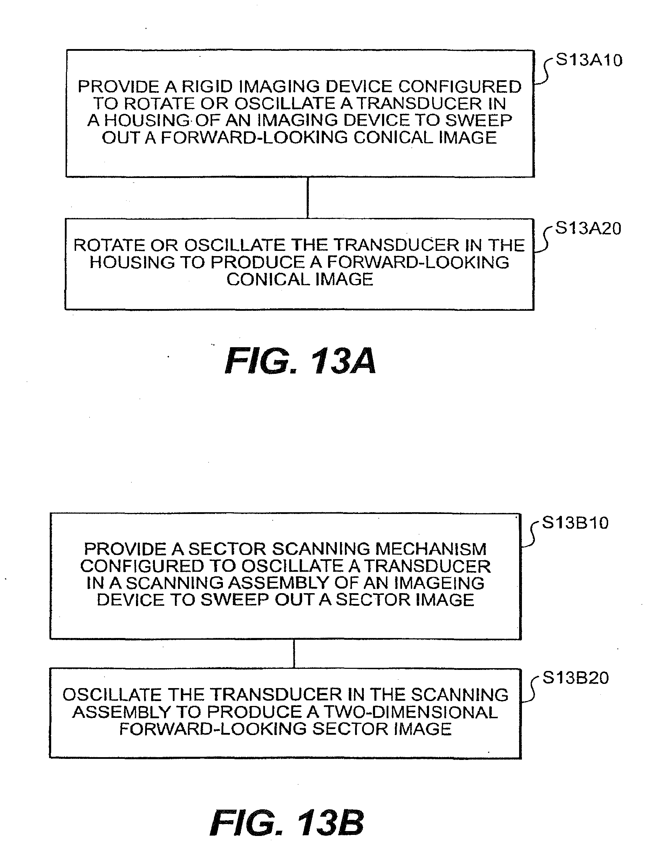

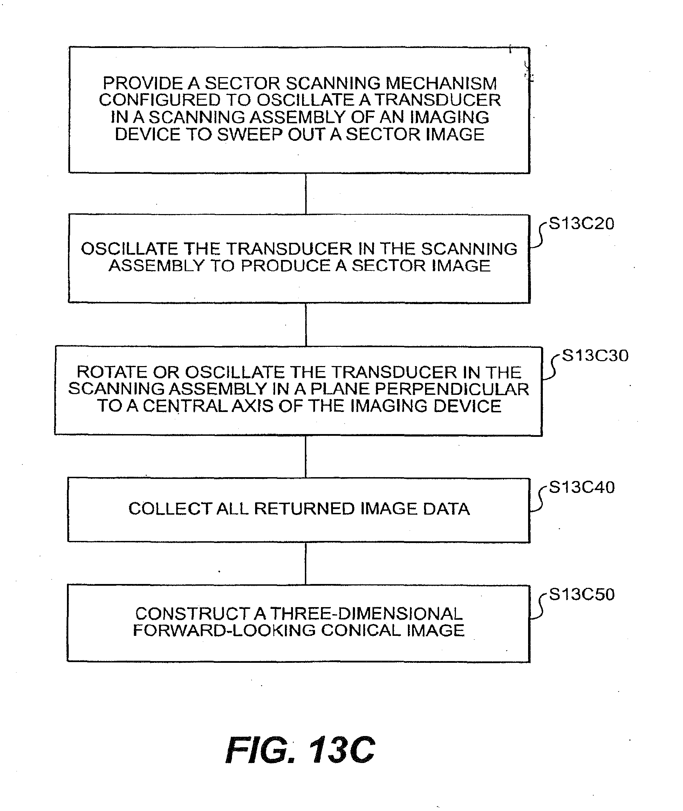

One exemplary embodiment of an imaging device configured for volume scanning is shown in Figure 12. The imaging device 1200 of Figure 13 may be in the form of an imaging needle assembly. Further, the imaging device 1200 shown in Figure 12 may include a scanning assembly 1266 mounted within housing 1230. The sector scanning mechanism 1290 may be driven by motor 1296. That is, output shaft 1291 may be attached to connector mechanism 1297 via drive shaft 1299a. Drive shafts 1299a and 1299b and connector mechanism 1297 also connect the motor 1296 to gear 1298a which mates with gear 1298b mounted on housing 1230 to rotate the imaging device 1200 in a rotary motion.

Figures 13A-13C are flow charts of a method of imaging using an imaging device according to embodiments. The method of Figure 13A includes providing a rigid imaging device configured to rotate or oscillate a transducer in a housing of the imaging device, while recording changes in an angle of the housing with respect to a patient, to sweep out a forward-looking conical image (step 13A10). The transducer is then rotated or oscillated in the housing to produce a forward-looking conical image (step 13A20). The rigid imaging device may be, for example, a rigid imaging needle device, a rigid imaging drill device, or a rigid imaging reamer device.

The method of Figure 13B includes providing a sector scanning mechanism configured to oscillate a transducer in a scanning assembly of an imaging device to sweep out a sector image (step 13B10). The transducer is then oscillated in the scanning assembly to produce a two-dimensional forward-looking sector image (step 13B30).

The method of Figure 13C includes providing a sector scanning mechanism configured to oscillate a transducer in a scanning assembly of an imaging device to sweep out a sector image (step 13C10). The transducer is oscillated in the scanning assembly to produce a sector image (step 13C20). Then, the transducer is rotated or oscillated in the scanning assembly in a plane perpendicular to a central axis of the imaging device, while changes in an angle of a housing of the imaging device with respect to a patient are recorded (step 13C30). All of the returned data is then collected (step 13C40), and a three-dimensional forward-looking conical image is constructed (step 13C50).

The angle of the scanning assembly with respect to the central axis of the imaging device may be adjustable within a range of ∼0° to ∼ 180°. More particularly, the angle of the scanning assembly with respect to the central axis of the imaging device may be adjustable within a range of ∼60° to ∼ 120°. Further, step 13C20 may be performed continuously during step 13C30. Furthermore, steps 13C20-13C30 may be configured to produce a spiral scan.

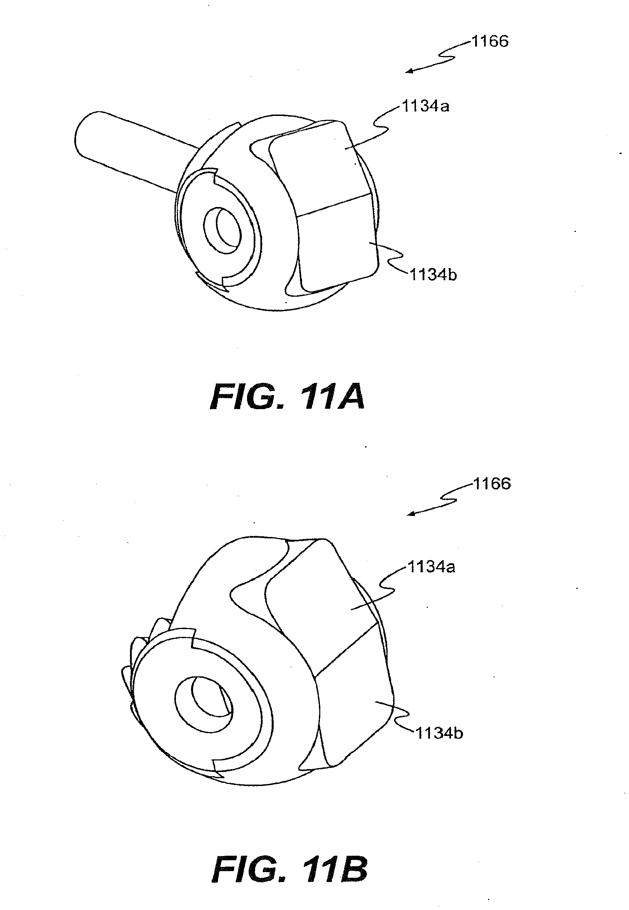

In each of the above discussed embodiments, the scanning assembly is shown to include one transducer. However, large ranges of motions may be difficult to achieve with only one transducer. The required range of motion may be cut in half by using, for example, two transducers, as shown in Figure 11A-11C. In the exemplary embodiment shown in Figure 11A-11B, the scanning assembly 1166 includes two transducers 1134a and 1134b, which may be placed at half of the sector angles and alternately fired to generate scan lines.

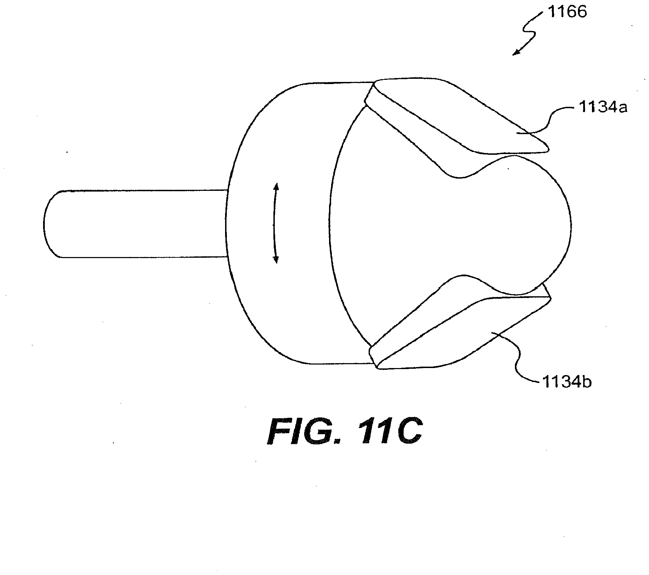

Further, in certain cases it may be beneficial to scan, instead of one conical forward-looking image, two or more conical forward looking images, where such information may be advantageous. FigurellC shows an embodiment in which two transducers are provided. In the embodiment of Figure 11C, the two transducers are positioned at different angles about the central longitudinal axis of the scanning assembly. The two transducers may be operated at different scanning frequencies to image different tissue types or structures. In Figures 11A-11C, two transducers are shown; however, more than two transducers may be utilized to produce proportional results.

Any reference in this specification to "one embodiment," "an embodiment," "example embodiment," etc., means that a particular feature, structure, or characteristic described in connection with the embodiment is included in at least one embodiment of the invention. The appearances of such phrases in various places in the specification are not necessarily all referring to the same embodiment. Further, when a particular feature, structure, or characteristic is described in connection with any embodiment, it is submitted that it is within the purview of one skilled in the art to effect such feature, structure, or characteristic in connection with other ones of the embodiments.

The invention further relates to an imaging device, comprising: a housing; a scanning assembly configured to output signals and receive return signals to produce an image when oscillated, wherein the scanning assembly includes an ultrasonic transducer disposed within the housing at an angle with respect to a central axis of the imaging device; and an angle encoder configured to encode a rotational angle of the scanning assembly, the device further comprising a sector scanning mechanism configured to rotate the scanning assembly about the central axis of the imaging device to produce a sector image.

Preferably, the sector scanning mechanism comprises: a rotatable axle on which the scanning assembly including the transducer is mounted; and a drive shaft configured to rotate the axle. The sector scanning mechanism may further comprise a gear configured to convert rotation of the drive shaft into angular displacement of the transducer. The sector scanning mechanism may further comprise a slotted encoder wheel configured to record an angle of the scanning assembly. The sector scanning mechanism may further comprise a support mechanism configured to support the drive shaft within the housing. The sector scanning mechanism may further comprise an angle encoder attached to the drive shaft, wherein the angle encoder may comprise a slit wheel and a photo interrupter. The sector scanning mechanism may comprise a draw string mechanism configured to rotate the axle. The draw string mechanism may comprises a pulley mounted on a rotatable shaft and a draw string attached to the pulley and to the scanning assembly. The imaging device may further comprise an angle encoder attached to the rotatable shaft. In a further embodiment the sector scanning mechanism may comprise: a tube disposed within the housing; at least one slit formed in one end of the tube adjacent to the scanning assembly; and at least one pin provided on the scanning assembly to mate with the at least one slit. The sector scanning mechanism may further comprise a gear provided at another end of the tube. The sector scanning mechanism may further comprise an angle encoder attached to the tube. The sector scanning mechanism may comprise a pull-and-retract mechanism. The pull-and-retract mechanism may comprise: a cable or rod attached to the scanning assembly; a pulley attached to the cable or rod; and a restoring spring attached to the scanning assembly.

In a further embodiment, the imaging device as is set forth in claim 1 may further comprise a sector scanning mechanism configured to oscillate the scanning assembly to scan a sector. In the imaging device the sector scanning mechanism may oscillate the scanning assembly in a plane substantially perpendicular to the central longitudinal axis of the scanning assembly to produce a sector image. The sector scanning mechanism may comprise an oscillating drive shaft mechanism. The oscillating drive shaft mechanism may comprise: an input shaft; an input crank attached to the input shaft; a connecting rod one end of which is attached to the input shaft; an output crank attached to the other end of the connecting rod; and an output shaft attached to the output crank and to the scanning assembly. In a further embodiment, the oscillating drive shaft mechanism may further comprise a drive mechanism attached to the input shaft. The imaging device may further comprise a rotating mechanism configured to rotate the housing. Ina still further embodiment of the imaging device the rotation mechanism may comprise: a first gear mounted on the housing; a second gear configured to drive the first gear; and a drive shaft attached to the second gear. The imaging device may further comprise a drive mechanism attached to the drive shaft. The drive mechanism may be configured to drive both the rotating mechanism and the sector scanning mechanism. In a further embodiment of the imaging device the sector scanning mechanism may comprise a input shaft; an input crank attached to the input shaft; a connecting rod one end of which is attached' to the input shaft; an output crank attached to the other end of the connecting rod; and an output shaft attached to the output crank and to the scanning assembly. The imaging device may further comprise a connection mechanism configured to connect the drive mechanism to the drive shaft of the rotating mechanism and the input shaft of the sector scanning mechanism, wherein the sector scanning mechanism may comprise an oscillating motor.

In a still further embodiment of the imaging device as is recited in claim 1, the scanning assembly may comprise at least two transducers mounted thereon. Preferably, the at least two transducers are each mounted at an angle about the central longitudinal axis of the scanning assembly. In an embodiment of the imaging device the at least two transducers are mounted at different angles about the central longitudinal axis of the scanning assembly, such that the scanning assembly produces at least two images when oscillated. In a further embodiment of the imaging device the at least two transducers are mounted at different angle from the central axis of the imaging device, such that two scan cones are generated. The at least two transducers may be mounted at different locations along the central axis of the imaging device. In a still further embodiment of the imaging device, the at least two transducers are mounted adjacent to one another in a plane extending substantially perpendicular to the central longitudinal axis of the scanning assembly, thereby increasing an angular range of the scanning assembly and reducing an operating range of the scanning assembly. In a still further embodiment of the imaging device the at least two transducers are configured to be operated at different scanning frequencies to image different tissue types or structures.

It will be appreciated that the imaging device according to the invention may be used to facilitate a method of placing a tip of a diagnostic or therapeutic assembly at a specific location in tissue, comprising forming an image using an external imaging system; advancing an imaging device toward a general area of tissue with guidance from the image produced by the external imaging system; advancing the imaging device to a precise location guided by an image produced using signals from the imaging device; and performing a diagnostic or therapeutic procedure at the location. In an embodiment the method may further comprise: advancing a pre-loaded diagnostic or therapeutic assembly co-axially over the imaging device; and withdrawing co-axially the imaging device from a lumen of the diagnostic or therapeutic assembly, wherein performing a diagnostic or therapeutic procedure at the location comprises performing a diagnostic or therapeutic procedure at the location using the diagnostic or therapeutic assembly. The method as is set forth in the foregoing may be used to perform a diagnostic procedure. In a further embodiment of the method the diagnostic procedure may be one of a general biopsy procedure, a breast biopsy procedure, a prostate biopsy procedure, an aspiration procedure, an amniocentesis procedure, a cordocentesis procedures, or a transabdominal chorionic villus sampling procedure. In a still further embodiment of the method, it may be employed to perform a therapeutic procedure. In particular, the therapeutic procedure may be one of RF ablation, a chemical injection, or a brachytherapy seed placement procedure. The method as is set forth in the foregoing may be practiced on a lesion to be diagnosed or treated is less than ∼3 millimeters in size.

In accordance to a further embodiment, a method of placing a tip of a diagnostic or therapeutic assembly at a specific location in tissue, comprises: palpating a suspect lesion; advancing an imaging device toward a general area of tissue based on a general location of the palpated lesion; advancing the imaging device to a precise location of the palpated lesion guided by images produced using signals from the imaging device; and performing a diagnostic or therapeutic procedure at the location. The method may further comprise: advancing a pre-loaded diagnostic or therapeutic assembly co-axially over the imaging device; withdrawing co-axially the imaging device from a lumen of the diagnostic or therapeutic assembly, - wherein performing a diagnostic or therapeutic procedure at the location comprises performing a diagnostic or therapeutic procedure at the location using the diagnostic or therapeutic assembly. The method may be employed to perform a diagnostic procedure. In particular, the diagnostic procedure may be one of a general biopsy procedure, a breast biopsy procedure, a prostate biopsy procedure, an aspiration procedure, an amniocentesis procedure, a cordocentesis procedure, or a transabdominal chorionic villus sampling procedure. Alternatively or additionally, the method may be employed to perform a therapeutic procedure. In particular, the therapeutic procedure is one of a RF ablation, a chemical injection, or a brachytherapy seed placement procedure. A lesion to be diagnosed or treated may be less than ∼3 millimeters in size.

Another aspect of the invention relates to a method of imaging using an imaging device, the method comprising: providing a rigid imaging device configured to rotate or oscillate a transducer in a housing of the imaging device, while recording changes in an angle of the housing with respect to a patient, to sweep out a forward-looking conical image; and rotating or oscillating the transducer in the housing to produce a forward- looking conical image. In the method the rigid imaging device may be one of a rigid imaging needle device, a rigid imaging drill device, or a rigid imaging reamer device.

Still another aspect of the invention relates to a method of imaging using an imaging device having a scanning assembly, the method comprising: a) providing a sector scanning mechanism configured to oscillate a transducer in the scanning assembly to sweep out a sector image; and b) oscillating the transducer in the scanning assembly to produce a sector image. The method may further comprise: c) rotating or oscillating the transducer in the scanning assembly in a plane perpendicular to a central axis of the imaging device, while recording changes in an angle of a housing of the imaging device with respect to a patient; d) collecting all returned image data; and e) constructing a three-dimensional forward-looking conical image. In the method maximum and minimum angles of the scanning assembly with respect to the central axis of the imaging device may be adjustable within a range of ∼0# to ∼ 180°. In the method maximum and minimum angles of the scanning assembly with respect to the central axis of the imaging device may be adjustable within a range of -60° to ∼ 120°. In the method step b) may be performed continuously during step c). In the steps b)-c) may produce a spiral scan.

Although embodiments have been described with reference to a number of illustrative embodiments thereof, it should be understood that numerous other modifications and embodiments can be devised by those skilled in the art that will fall within the spirit and scope of the principles of this invention. More particularly, reasonable variations and modifications are possible in the component parts and/or arrangements of the subject combination arrangement within the scope of the foregoing disclosure, the drawings and the appended claims without departing from the spirit of the disclosure. In addition to variations and modifications in the component parts and/or arrangements, alternative uses will also be apparent to those skilled in the art.

## Claims

1. A method of assembling a needle-based imaging device, comprising:
providing a needle housing having a central axis; and
disposing a scanning assembly within a distal portion of the needle housing at an oblique angle with respect to the said central axis such that the ultrasonic transducer is configured to provide a forward-looking image with respect to the needle housing;
connecting a scanning assembly to the needle housing, wherein the scanning assembly is configured to output signals and to receive return signals for producing an image when oscillated in use.

2. The method of claim 1, further comprising: connecting an angle encoder to the needle housing, the angle encoder configured to encode a rotational angle of the scanning assembly.

3. The method of any of the preceding claims, wherein the ultrasonic transducer is configured to provide a conical forward-looking image.

4. The method of any of the preceding claims, wherein the needle housing includes at least one grip disposed on an outer surface of the needle housing

5. The method of any of the preceding claims, wherein the ultrasonic transducer is fixedly attached to the needle housing such that the ultrasonic transducer rotates with the needle housing.

6. The method of any of the preceding claims, wherein the ultrasonic transducer is disposed at an oblique angle between about 10° and about 30° with respect to the central axis of the needle housing.

7. The method of any of claims 1 or 2, wherein the ultrasonic transducer is configured to provide a forward-looking sector image.

8. The method of claim 7, wherein the ultrasonic transducer is coupled to a drive mechanism that oscillates the ultrasonic transducer relative to the needle housing.

9. The method of claim 7, wherein the ultrasonic transducer is coupled to a sector scanning mechanism configured to rotate the ultrasonic transducer about the central axis of the needle housing to produce a sector image.

10. The method of any of the preceding claims, wherein a distal end of the provided needle housing is sharp.

11. The method of any of claims 1-8, wherein a distal end of the provided needle housing is blunt.

12. The method of any of the preceding claims, further comprising attaching a therapy device to the distal portion of the needle housing.

13. The method of claim 12, wherein the therapy device is an ablation device, a biopsy device or a drainage device.

14. A needle-based imaging device, comprising:
- a needle housing having a central axis;
- a scanning assembly disposed within a distal portion of the needle housing at an oblique angle with respect to the said central axis such that the ultrasonic transducer is configured to provide a forward-looking image with respect to the needle housing;
- a scanning assembly connected to the needle housing, wherein the scanning assembly is adapted to oscillate in use and is configured to output signals and to receive return signals for producing an image.

15. The needle-based imaging device according to claim 14, wherein the ultrasonic transducer is fixedly attached to the needle housing such that the ultrasonic transducer rotates with the needle housing.
